# EUROPEAN PATENT APPLICATION

(11) **EP 4 732 787 A2**
(43) Date of publication of application: **29.04.2026**
(21) Application number: 26165242.4
(22) Date of filing: 08.02.2023
(51) Int. Cl.: A61B 17/12

(54) **VASO-OCCLUSIVE DEVICE AND DELIVERY ASSEMBLY**

(30) Priority: 11.02.2022 US 202263309300 P
(62) Divisional of application: 23718546.7
(71) Applicant: STRYKER CORPORATION, Portage, MI 49002-9711 (US); Stryker European Operations Limited, Carrigtwohill, Co. Cork T45 HX08 (IE)
(72) Inventor: CHEN, Hancun, San Ramon, 94582 (US); LEE, Andrew S., San Jose, 95124 (US); DAO, Jimmy, Kalamazoo, 49002 (US); GAMARRA, Randy, Kalamazoo, 49002 (US); TRAN, Kevin, Kalamazoo, 49002 (US)
(74) Representative: Gillespie, Richard

(57) **Abstract**

A vaso-occlusive treatment system includes a delivery assembly and a vaso-occlusive device detachably coupled to the delivery assembly by a delivery assembly junction. The vaso-occlusive device includes a braided portion formed out of one or more wires, the braided portion including a packed end bundle. The vaso-occlusive device also includes a coiled portion coupled to the braided portion. The vaso-occlusive device further includes an intra-device junction coupling the braided portion to the coiled portion, the intra-device junction including a stretch-resistant member spanning from the packed end bundle to the delivery assembly junction.

## Description

### Cross Reference to Related Application

This application claims the benefit of U.S. Provisional Patent Application Serial No. 63/309,300, filed on February 11, 2022, the entire disclosure of which is expressly incorporated by reference herein.

### Field

The field of the disclosure generally relates to vaso-occlusive devices for establishing an embolus or vascular occlusion in a vessel of a human patient. More particularly, the disclosure relates to at least partially braided or woven vaso-occlusive devices, junctions within such devices and junctions for coupling such devices to treatment systems.

### Background

Vaso-occlusive devices or implants are used for a wide variety of reasons, including treatment of intra-vascular aneurysms. Commonly used vaso-occlusive devices include soft, helically wound coils formed by winding a platinum (or platinum alloy) wire strand about a "primary" mandrel. The coil is then wrapped around a larger, "secondary" mandrel, and heat treated to impart a secondary shape. For example, U.S. Pat. No. 4,994,069, issued to Ritchart et al., which is fully incorporated herein by reference as though set forth in full, describes a vaso-occlusive device that assumes a linear, helical primary shape when stretched for placement through the lumen of a delivery catheter, and a folded, convoluted secondary shape when released from the delivery catheter and deposited in the vasculature. Other examples of vaso-occlusive devices include at least partially braided or woven devices, such as those described in U.S. Pat. No. 10,321,915, issued to Murphy et al., and U.S. Pat. No. 10,893,870, issued to Wang et al., which are fully incorporated herein by reference as though set forth in full.

In order to deliver the vaso-occlusive devices to a desired site in the vasculature, e.g., within an aneurysmal sac, it is well-known to first position a small profile, delivery catheter or "micro-catheter" at the site using a steerable guidewire. Typically, the distal end of the micro-catheter is provided, either by the attending physician or by the manufacturer, with a selected pre-shaped bend, e.g., 45°, 90°, "J", "S", or other bending shape, depending on the particular anatomy of the patient, so that it will stay in a desired position for releasing one or more vaso-occlusive device(s) into the aneurysm once the guidewire is withdrawn. A delivery or "pusher" assembly or "wire" is then passed through the micro-catheter, until a vaso-occlusive device pushed by a distal end of the delivery assembly is extended out of the distal end opening of the micro-catheter and into the aneurysm. Once in the aneurysm, portions of the vaso-occlusive device deform or bend to allow more efficient and complete packing. Vaso-occlusive devices that are coupled to the distal end of the delivery assembly are released or "detached" from the distal end of the delivery assembly, after extending into the aneurysm. Then, the delivery assembly is withdrawn back through the catheter. Depending on the particular needs of the patient, one or more additional vaso-occlusive devices may be pushed through the catheter and released at the same site.

One well-known way to release a vaso-occlusive device from the end of the delivery assembly is through the use of an electrolytically severable junction, which is a small exposed section or detachment zone located along a distal end portion of the delivery assembly. The detachment zone is typically made of stainless steel and is located just proximal of the vaso-occlusive device. An electrolytically severable junction is susceptible to electrolysis and disintegrates when the delivery assembly is electrically charged in the presence of an ionic solution, such as blood or other bodily fluids. Thus, once the detachment zone exits out of the catheter distal end and is exposed in the vessel blood pool of the patient, a current applied through an electrical contact to the conductive pusher completes an electrolytic detachment circuit with a return electrode, and the detachment zone disintegrates due to electrolysis. Other detachment mechanisms for releasing a vaso-occlusive device from a delivery assembly include mechanical, thermal, and hydraulic mechanisms.

In order to better frame and fill aneurysms, complex three-dimensional secondary shapes can be imparted on vaso-occlusive devices and the stiffness/flexibility of vaso-occlusive devices can be modified. However, vaso-occlusive devices continue to have performance limitations including breaking performance, shape retention and flexibility.

The proximal end of some vaso-occlusive devices is coupled to the distal end of the delivery assembly with what is known as a "main junction" of the vaso-occlusive treatment system, or a "delivery assembly junction" or simply a "delivery junction." Another main junction design is disclosed in U.S. Pat. No. 8,202,292, issued to Kellett, which is fully incorporated herein by reference as though set forth in full. The main junction includes a flat adapter coupling a delivery wire to a vaso-occlusive device. The delivery wire has a hook or "J" shape distal end configured to be received in an aperture in the proximal end of the adapter to couple the delivery wire to adapter. The vaso-occlusive device has windings that define openings configured to receive fingers in the distal end of the adapter to couple the vaso-occlusive device to the adapter. Consequently, the adapter facilitates coupling of the delivery wire to the vaso-occlusive device. Other main junction designs are disclosed in U.S. Pat. No. 9,480,479, issued to Chen et al., which is fully incorporated herein by reference as though set forth in full.

Some vaso-occlusive devices include one or more braided portions and one or more coiled portions. In such vaso-occlusive devices, the braided and coiled portions are coupled by one or more "intra-device junctions". Some vaso-occlusive devices also include stretch-resistant members configured to keep coiled portions compacted or compressed to control the structural characteristics (e.g., flexibility and stiffness) of the coiled portions, which can change when coiled portions are stretched. Stretch-resistant members can form portions of both intra-device junctions and delivery junctions. In some vaso-occlusive devices, stretch-resistant members are formed from one or more braid wires, which are threaded from a first end of and through a coiled portion and secured at a second end thereof. In such vaso-occlusive devices, the diameters of stretch-resistant members depend on the diameters of the braid wires forming the braided portions. Accordingly, structural characteristics of intra-device junctions and delivery junctions are unnecessarily dependent on characteristics of the braid wires. Consequently, vaso-occlusive devices having braided portions with fine braid wires (e.g., diameters of 0.0008 in. or 0.0009 in.) will have intra-device junctions and delivery junctions with lower tensile strength.

Accordingly, there remains a need for vaso-occlusive treatment systems having vaso-occlusive devices where the size of braided portions is decoupled from the strength of intra-device junctions and delivery junctions, and methods for manufacturing same.

### Summary

According to one aspect of the disclosure, a vaso-occlusive treatment system includes a delivery assembly and a vaso-occlusive device detachably coupled to the delivery assembly by a delivery assembly junction. The vaso-occlusive device includes a braided portion formed out of one or more wires, the braided portion including a packed end bundle. The vaso-occlusive device also includes a coiled portion coupled to the braided portion. The vaso-occlusive device further includes an intra-device junction coupling the braided portion to the coiled portion, the intra-device junction including a stretch-resistant member spanning from the packed end bundle to the delivery assembly junction.

According to various aspects of the disclosure, the packed end bundle includes an adhesive disposed at an end of the braided portion or a weld disposed at an end of the braided portion. The packed end bundle may define an end surface that is orthogonal or oblique to a longitudinal axis of the packed end bundle.

According to various aspects of the disclosure, the stretch-resistant member includes first and second ends. The first end of the stretch-resistant member may terminate in the delivery wire junction, and the second end of the stretch-resistant member may terminate within the braided portion or the coiled portion. The first and second ends may be flattened. The first and second ends may each be terminated within the braided portion. The first and second ends may cross each other within the braided portion.

According to various aspects of the disclosure, the stretch-resistant member includes a bend. The first and second ends may terminate within the delivery wire junction, and the bend may be disposed adjacent the packed end bundle. The bend may be disposed distal of the packed end bundle. The first and second ends may terminate adjacent the packed end bundle, and the bend may be disposed within the delivery wire junction. The delivery assembly junction may include a link defining an opening, and the bend may be disposed in the opening. Each of the first and second ends may be terminated within the braided portion.

According to various aspects of the disclosure, the vaso-occlusive treatment system also includes a second stretch-resistant member, and the second stretch-resistant member includes third and fourth ends. The first and third ends may terminate within the delivery wire junction, and the second and fourth ends may terminate adjacent the packed end bundle.

According to various aspects of the disclosure, the stretch-resistant member is a loop including first and second bends. The first bend may be disposed within the delivery wire junction, and the second bend may be disposed adjacent the packed end bundle. The first bend may be disposed distal of the packed end bundle. The delivery assembly junction may include a link defining an opening, and the second bend may be disposed in the opening.

According to various aspects of the disclosure, the packed end bundle is a proximal end bundle, the coiled portion is a proximal coiled portion, the intra-device junction is a proximal intra-device junction, the stretch-resistant member is a proximal stretch-resistant member, and the braided portion further includes a distal packed end bundle. The system also includes a distal coiled portion coupled to the distal packed end bundle and a distal intra-device junction coupling the braided portion to the distal coiled portion. The distal intra-device junction includes a distal stretch-resistant member spanning from the distal packed end bundle to a distal end of the distal coiled portion.

According to various aspects of the disclosure, the distal packed end bundle includes an adhesive disposed at a distal end of the braided portion or a weld disposed at a distal end of the braided portion. The distal packed end bundle may define a distal end surface that is orthogonal or oblique to a longitudinal axis of the distal packed end bundle.

According to various aspects of the disclosure, the distal stretch-resistant member includes first and second distal stretch-resistant member ends. The first distal stretch-resistant member end of the distal stretch-resistant member may terminate in the distal end of the distal coiled portion, and the second distal stretch-resistant member end of the distal stretch-resistant member may terminate within the braided portion or the distal coiled portion. The first and second distal stretch-resistant member ends may be flattened. The first and second distal stretch-resistant member ends may each be terminated within the braided portion. The first and second distal stretch-resistant member ends may cross each other within the braided portion.

According to various aspects of the disclosure, the distal stretch-resistant member includes a distal stretch-resistant member bend. The first and second distal stretch-resistant member ends may terminate within the distal end of the distal coiled portion, and the distal stretch-resistant member bend may be disposed adjacent the distal packed end bundle. The distal stretch-resistant member bend may be disposed proximal of the distal packed end bundle. The first and second distal stretch-resistant member ends may terminate adjacent the distal packed end bundle, and the distal stretch-resistant member bend may be disposed within the distal end of the distal coiled portion. Each of the first and second distal stretch-resistant member ends may be terminated within the braided portion.

According to various aspects of the disclosure, the vaso-occlusive treatment system also includes a second distal stretch-resistant member, and the second distal stretch-resistant member includes third and fourth distal stretch-resistant member ends. The first and third distal stretch-resistant member ends may terminate within the distal end of the distal coiled portion, and the second and fourth distal stretch-resistant member ends may terminate adjacent the distal packed end bundle.

According to various aspects of the disclosure, the distal stretch-resistant member is a loop including first and second distal stretch-resistant member bends. The first distal stretch-resistant member bend may be disposed within the distal end of the distal coiled portion, and the second distal stretch-resistant member bend may be disposed adjacent the distal packed end bundle. The first distal stretch-resistant member bend may be disposed proximal of the distal packed end bundle.

According to various aspects of the disclosure, the delivery assembly has a distal end, where the distal end forms a hook. The delivery assembly junction may include a link having a proximal end and a distal end, where the distal end of the link includes a plurality of fingers.

According to another aspect of the disclosure, a method of manufacturing a vaso-occlusive treatment system includes forming a vaso-occlusive device. Forming the vaso-occlusive device includes disposing a tubing around a proximal end of a braided portion formed from one or more wires, applying heat to the tubing to shrink the tubing around the proximal end of the braided portion to form a shrunken proximal end, applying glue to the shrunken proximal end to form a packed end bundle, removing the tubing from around the packed end bundle, threading a stretch-resistant member through the braided portion adjacent to and distal of the packed end bundle to form a bend of the stretch-resistant member in the braided portion adjacent to and distal of the packed end bundle, inserting the stretch-resistant member and at least a portion of the packed end bundle into a coiled portion, and applying glue to the packed end bundle and the bend of the stretch-resistant member to form an intra-device junction. The method also includes coupling a distal end of a delivery assembly to a proximal end of the coiled portion and a proximal end of the stretch-resistant member. The method further includes applying glue to the distal end of the delivery assembly, the proximal end of the coiled portion, and the proximal end of the stretch-resistant member to form a delivery junction.

According to still another aspect of the disclosure, a method of manufacturing a vaso-occlusive treatment system includes forming a packed end bundle. Forming the packed end bundle includes disposing a tubing around a proximal end of a braided portion formed from one or more wires, applying heat to the tubing to shrink the tubing around the proximal end of the braided portion to form a shrunken proximal end, applying glue to the shrunken proximal end to form a packed end bundle, and removing the tubing from around the packed end bundle. The method also includes forming a delivery junction. Forming the delivery junction includes coupling a distal end of a link to a proximal end of a coiled portion, coupling a distal end of a delivery assembly to a proximal end of the link, threading a stretch-resistant member through an opening defined by the link to form a bend of the stretch-resistant member in the opening, and applying glue to the link, the proximal end of the coiled portion, the distal end of the delivery assembly, and the bend of the stretch-resistant member to form the delivery junction. The method further includes inserting at least a portion of the packed end bundle into the coiled portion. Moreover, the method includes coupling an end of the stretch-resistant member to the braided portion adjacent to and distal of the packed end bundle. In addition, the method includes applying glue to the packed end bundle and the end of the stretch-resistant member to form an intra-device junction.

According to various aspects of the disclosure, the coiled portion is a proximal coiled portion, and the method also includes coupling a distal coiled portion to a distal end of the braided portion.

Other and further aspects and features of the disclosure will become apparent from the ensuing detailed description in view of the accompanying figures.

### Brief Description of the Drawings

This patent or application file contains at least one drawing executed in color. Copies of this patent or patent application publication with color drawings will be provided by the U.S. Patent and Trademark Office upon request and payment of the necessary fee.

The drawings illustrate the design and utility of various aspects of the disclosure, in which similar elements are referred to by common reference numerals. These drawings are not necessarily drawn to scale. In order to better appreciate how the above-recited and other advantages and objects are obtained, a more particular description of the disclosure will be rendered, which is illustrated in the accompanying drawings. These drawings depict only exemplary aspects of the disclosure for purposes of illustration and facilitating the below detailed description, and are not therefore to be considered limiting of its scope.
Figure 1 is a schematic view of an exemplary vaso-occlusive treatment system, including a vaso-occlusive device and a delivery assembly according to various aspects of the disclosure.
Figures 2A and 2B are detailed schematic views depicting steps in manufacturing an exemplary vaso-occlusive device according to various aspects of the disclosure.
Figures 3 to 7 are schematic views of exemplary vaso-occlusive treatment systems, including respective vaso-occlusive devices and delivery assemblies according to various aspects of the disclosure.
Figures 8 to 10 are detailed schematic views of exemplary intra-device junctions in vaso-occlusive devices according to various aspects of the disclosure.
Figures 11A to 11I are a series of schematic views depicting an exemplary method for manufacturing a vaso-occlusive treatment system, including a vaso-occlusive device and a delivery assembly according to various aspects of the disclosure.
Figures 12A to 12I are a series of schematic views depicting an exemplary method for manufacturing a vaso-occlusive treatment system, including a vaso-occlusive device and a delivery assembly according to various aspects of the disclosure.
Figures 13A to 13D are schematic views depicting proximal packed end bundles of respective vaso-occlusive devices according to various aspects of the disclosure.

### Detailed Description of the Disclosure

This specification describes exemplary aspects and applications of the disclosure. The disclosure, however, is not limited to these exemplary aspects and applications or to the manner in which the exemplary aspects and applications operate or are described herein. Further, the figures may show simplified or partial views, and the dimensions of elements in the figures may be exaggerated or otherwise not in proportion. Moreover, elements of similar structures or functions are represented by like reference numerals throughout the figures. In addition, an illustrated aspect needs not have all the features or advantages shown. A feature or an advantage described in conjunction with a particular aspect is not necessarily limited to that aspect, and can be practiced in any other aspect even if not so illustrated.

For the following defined terms, these definitions shall be applied, unless a different definition is given in the claims or elsewhere in this specification.

As the terms "on," "attached to," "connected to," "coupled to," "secured to" or similar words are used herein, one element (e.g., a material, a layer, a substrate, etc.) can be "on," "attached to," "connected to," "coupled to" or "secured to" another element regardless of whether the one element is directly on, attached to, connected to, coupled to or secured to the other element or there are one or more intervening elements between the one element and the other element. Directions (e.g., above, below, top, bottom, side, up, down, under, over, upper, lower, horizontal, vertical, "x," "y," "z," etc.), if provided, are relative and provided solely by way of example and for ease of illustration and discussion and not by way of limitation. Where reference is made to a list of elements (e.g., elements a, b, c), such reference is intended to include any one of the listed elements by itself, any combination of less than all of the listed elements, and/or a combination of all of the listed elements.

As used herein, "substantially" means sufficient to work for the intended purpose. The term "substantially" thus allows for minor, insignificant variations from an absolute or perfect state, dimension, measurement, result, or the like such as would be expected by a person of ordinary skill in the field but that do not appreciably affect overall performance. The term "ones" means more than one.

All numeric values are herein assumed to be modified by the term "about," whether or not explicitly indicated. The term "about" generally refers to a range of numbers that one of skill in the art would consider equivalent to the recited value (i.e., having the same function or result). In many instances, the terms "about" may include numbers that are rounded to the nearest significant figure. The recitation of numerical ranges by endpoints includes all numbers within that range (e.g., 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

As used herein, a plurality of "elongate members" used to form a braid can include only a single elongate member used to form the braid, i.e., wherein the single elongate member turns back on itself at the ends of the braid. As used herein, the terms "tube," "tubular," "diameter," "radius" and "circumference" encompass objects with non-circular cross section as well as those with circular cross sections. As used herein, the terms "stiffness" or "flexibility" as they pertain to wires, coils, braids, or portions thereof include, but are not limited to, bending stiffness. As used herein, the term "constrained" includes, but is not limited to: (1) a braid being held in a compressed, elongated configuration due to forces exerted thereon, such as by a delivery catheter; and (2) a dope being held in a compressed, shortened configuration due to forces exerted thereon, such as by a stretch-resistant member. As used herein, the term "unconstrained" includes, but is not limited to, the lack of external forces acting on objects, including but not limited to, braids and coils. While "unconstrained" is used synonymously with "deployed" herein for braids, the anatomy of some patients may exert forces on deployed vaso-occlusive devices, which may include braids.

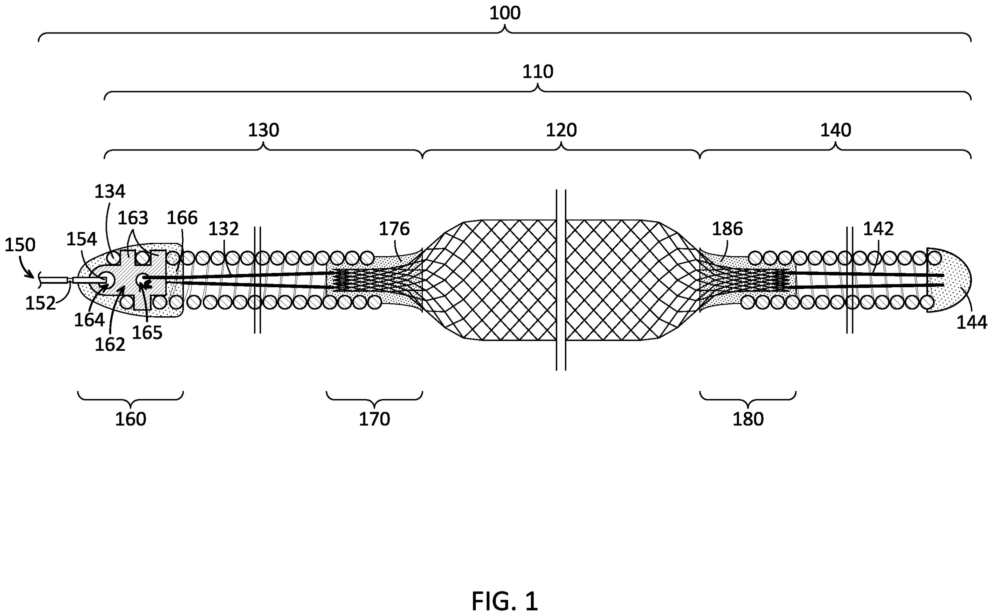

Figure 1 depicts a vaso-occlusive treatment system 100 including a vaso-occlusive device 110 having a braided portion 120 and proximal and distal coiled portions 130, 140. A proximal end of the vaso-occlusive device 110 is removably coupled to a delivery assembly 150 for delivery into a patient. Details regarding delivery wire assemblies and delivery of vaso-occlusive treatment systems are described in U.S. Pat. Nos. 8,202,292, 9,480,479, 10,321,915, and 10,893,870, which were previously incorporated by reference herein.

The braided portion 120 can be braided from any type of elongate members (e.g., "alloy wires", "wires"), as long as the material used to make such elongate members is biocompatible. For instance, the braided portion 120 can be braided from 24 wires, each wire having a cross-sectional diameter of 0.001 in. The braid can be a flat braid or a round braid. According to other aspects of the disclosure, the braided portion 120 can be braided from 16 to 32 wires, each wire having a cross-sectional diameter of 0.00075 in. to 0.0015 in. The proximal and distal portions 130, 140 are coils wound from one or more of the same or different alloy wires. According to other aspects of the disclosure, portion 120 can be braided from more than 32 wires, each wire having a cross-sectional diameter of 0.0.00075 in. to 0.0.00125 in.

According to some aspects of the disclosure, the alloy wires or the wires can be drawn filled tubing (DFT) available from Fort Wayne Metals in Fort Wayne, Indiana. The DFT wire includes a substantially pure Platinum ("Pt") core at least partially surrounded by a substantially pure Nitinol ("NiTi") external layer. As used in this application, "substantially pure" Pt includes but is not limited to 99.95% commercial purity produced in accordance with ASTM B561. The Pt core is approximately 40%-50% of the DFT wire by volume ("Pt core content"). According to other aspects of the disclosure, the DFT wires are formed by inserting a core component (a solid elongate member) into an external component (a tubular elongate member) to form a composite elongate member (e.g., a composite wire). The composite elongate member is repeatedly mechanically drawn and annealed by heating to increase its axial length and decrease its cross-sectional diameter. For example, the drawing and annealing process can serially reduce the diameter of a composite elongate member from 1 in. to 0.5 in., from 0.5 in. to 0.25 in., from 0.25 in. to 0.025 in., and from 0.025 in. to 0.0025 in.

While the materials (e.g., Pt and NiTi) forming various portions (e.g., core and external layer) of the DFT wire may have different stiffnesses (i.e., bending stiffness), the relative stiffnesses (i.e., bending stiffness) of the resulting portions of the DFT may not necessarily reflect the stiffnesses of the material from which they are made. For instance, even though Pt's bending modulus (i.e., stiffness) is greater than that of NiTi, Pt's bending modulus in combination with the Pt wire's diameter results in a platinum wire that is softer (i.e., less stiff) than the corresponding NiTi external layer.

The braided portion 120 of the vaso-occlusive device 110 can be braided on a mandrel, which can be flat or round depending on the desired final shape. After braiding, the braided portion 120 can be heat set (e.g., at 500 °C to 550 °C for 1 to 10 minutes). The heat set completed braid forms the linear "primary shape" of the braided portions 120. The heat set completed braid can then be wrapped around a second mandrel (e.g., a three-dimensional mandrel) and heat set for a second time to impart a three-dimensional "secondary shape." The NiTi external layer improves retention of the secondary shape.

While vascular implants (i.e., stents) have been formed from NiTi-DFT-Pt wires where the Pt core content is up to around 30%, NiTi-DFT-Pt wires with Pt core content around 40% to 50% have not been used to form stents. This is because stents typically require wires having higher yield strength to ultimate strength ratios (i.e., >80% of ultimate tensile strength ("UTS")) to maintain vessel patency. On the other hand, vaso-occlusive device 110 includes DFT wires with lower yield strength to UTS ratios, which improves device characteristics including lower bending moments and improved breaking performance. According to various aspects of the disclosure, the DFT wires that form (portions of) the vaso-occlusive device 110 have yield strength to UTS ratios of <50% UTS, <60% UTS, <70% UTS and <80% UTS. Yield strength is the maximum amount of force which can be applied to a material before it begins to plastically deform. UTS is the minimum amount of force which must be applied to a material before it fails. Braiding at least a portion of a vaso-occlusive device from wires having a yield strength to UTS ratio of less than 80% UTS is critical to simultaneously achieving the characteristics of improved radiopacity, shape retention and breaking performance.

According to other aspects of the disclosure, vascular implants, including vaso-occlusive devices, are formed from NiTi-DFT-Pt wires with Pt core content of around 40% to 50%. Braiding the braided portion 120 from DFT wires with Pt core content of around 40% to 50% ("NiTi-DFT-40/50Pt") provides a braided portion 120 that has (1) radiopacity throughout its entire length, (2) improved shape retention, and (3) improved breaking performance along the entire length of the braid. The Pt core provides the radiopacity for a substantial proportion of the vaso-occlusive device 110, providing radiopacity of various vaso-occlusive devices implanted into patients. The superelastic properties of the NiTi external layer contribute to the improved shape retention. The softness of the Pt contributes to the improved breaking performance, i.e., the ability of the vaso-occlusive device 110 to bend and fold to conform to the shape of body cavities. These characteristics of the braided portion 120 results in a vaso-occlusive device 110 that is more suitable for intra-saccular embolization of vascular defects, such as aneurysms, i.e., substantially consistent visualization grey scale throughout the vaso-occlusive device 110 and optimal softness profile.

Braiding at least a portion of a vaso-occlusive device 110 from 16 to 32 DFT wires, each wire having a cross-sectional diameter of 0.00075 in. to 0.0015 in. and having a Pt content of 35% to 60% simultaneously provides the characteristics of improved radiopacity, shape retention and breaking performance. As the Pt content is increased, the effect of the increasing Pt content on decreasing flexible is reduced. Accordingly, braids woven from DFT wires having Pt content of 35% to 60% are surprisingly suitable for vaso-occlusive applications, e.g., endo-saccular applications requiring flexible devices. Such braids are especially well suited when they are woven from 24 to 32 DFT wires, each wire having a cross-sectional diameter of 0.00075 in. to 0.00125 in. According to various aspects of the disclosure, at least a portion of a vaso-occlusive device 110 is braided from 24 to 32 DFT wires, each wire having a cross-sectional diameter of 0.00075 in. to 0.00125 in. and having a Pt content of 40% to 50%.

Normally the austenite finish, or "A_{f}" temperature of the NiTi is around 25°C, which is the temperature where the martensitic phase completes its transformation into the austenitic phase. Modifying the NiTi in the DFT such that its A_{f} temperature is between 30°C and 45°C results in a softer vaso-occlusive device. Setting the A_{f} temperature in this range achieves a desirable balance between device softness and conformability, which improves the suitability of the device for aneurysm treatment. Setting the A_{f} of the NiTi can be accomplished by adjusting the composition of Ni in NiTi from the normal of 50% to 50.4%-50.8%. Further, the A_{f} can be tuned with heat treatment of the NiTi. According to various aspects of the disclosure, the A_{f} temperature is between 38°C and 40°C.

The DFT can also include an oxide coating with a controlled thickness, which will enhance thrombogenisis (e.g., clotting) to increase vaso-occlusion within aneurysms. Preferably, the oxide coating has an average thickness between 50 nm and 500 nm. This is contrary to previous DFT braided implants (e.g., stents), from which oxide coatings are substantially removed (e.g., via electro-polishing), to generate "bright" stents. In previous DFT braided stents, oxide coating thickness is limited to less than 50 nm.

In other embodiments, instead of DFT wires, the braided portion 120 may be braided from elongate members formed from smaller DFT wires twisted together. Each DFT wire may be made of Niti-DFT-40Pt. Braiding elongate members (made from smaller DFT wires) instead of larger DFT wires results in a braided portion 120 that is softer at approximately the same Pt core content. Accordingly, the braided portion 120 according to this aspect of the disclosure (i.e., twisted elongate member braid) provides similar radiopacity with a softer braid. Such braids also provide higher surface area to promote thrombus formation, thereby enhancing aneurysm occlusion

The proximal and distal coiled portions 130, 140 are atraumatic coils to minimize tissue damage during insertion and deployment of the vaso-occlusive device 110. The proximal and distal coiled portions 130, 140 may be coils wound from one or more types of the wires such as nitinol wire, DFT, or other alloy wires.

The vaso-occlusive treatment system 100 includes a vaso-occlusive device 110 coupled to a delivery assembly 150 by a delivery junction 160. The delivery assembly 150 includes an electrolytically degradable segment 152 at a distal end thereof. The vaso-occlusive device 110 includes a braided portion 120 coupled to proximal and distal coiled portions 130, 140 by proximal and distal intra-device junctions 170, 180, respectively. The braided portion 120 is braided from alloy wires or other wires (for example, DFT) as described above, and has a substantially constant width (i.e., cross-sectional dimension). The proximal and distal coiled portions 130, 140 are respective coils wound from one or more alloy wires (such as DFT wires) as described above. According to various aspects of the disclosure, the braided and proximal and distal coiled portions 120, 130, 140 can be formed from any elongate members. The proximal coiled portion 130 also has a proximal stretch-resistant member 132, which extends distally from the delivery junction 160, through the proximal coiled portion 130, to the proximal intra-device junction 170, and reduces stretching of the proximal coil portion 130 as it is withdrawn proximally during delivery.

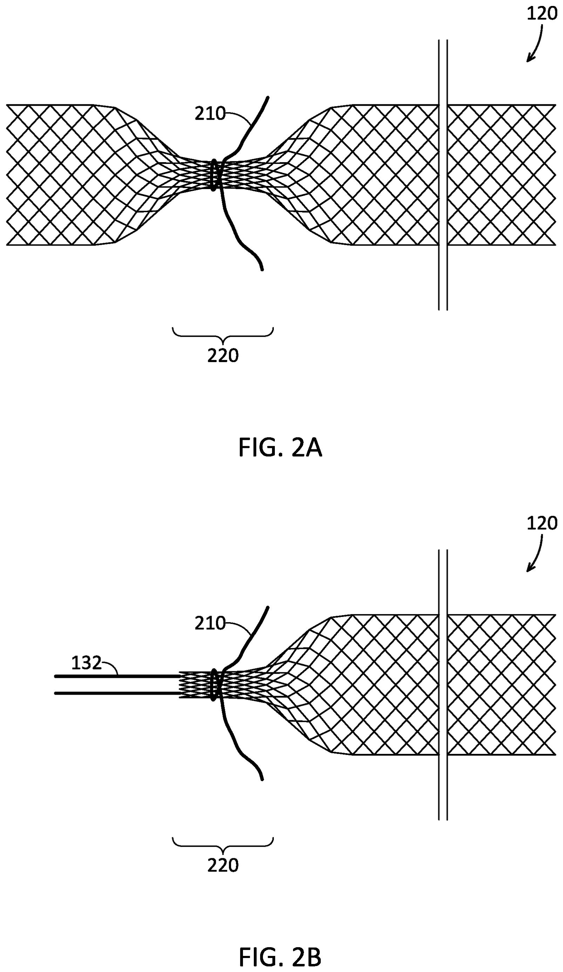

The proximal stretch-resistant member 132 is made from two of the braid wires as shown in Figures 2A and 2B. Figure 2A shows a first step in the manufacture of the braided portion 120. During manufacture, a tie down wire 210 is used to compress a necked down portion of the braided portion 120 to form a bundle 220. Figure 2B shows a second step in the manufacture of the braided portion 120, in which all of the braid wires except for two are trimmed off. The remaining two untrimmed braid wires are then used to form the proximal stretch-resistant member 132.

The delivery junction 160 includes a link/adapter 162 coupled to both the distal end of the delivery assembly 150 and the open proximal end of the proximal coiled portion 130, thereby coupling the delivery assembly 150 and the proximal coiled portion 130. The link 162 may be formed from a sheet, as described in U.S. Pat. No. 8,202,292, which were previously incorporated herein by reference herein. The link 162 is generally flat, and includes a plurality of fingers 163 formed in a proximal end thereof. The link also includes proximal and distal apertures 164, 165. The proximal coiled portion 130 of the vaso-occlusive device 110 is a coil with open pitch proximal windings 134 at the open proximal end thereof. At least some of these proximal windings 134 interlace with the fingers 163 of the link 162, coupling the link 162 to the proximal coiled portion 130 of the vaso-occlusive device 110.

The delivery junction 160 also includes a hook 154 formed from a distal end of a core wire of the delivery assembly 150, as described above. The hook 154 passes through the proximal aperture 164 of the link 162, coupling the delivery assembly 150 and link 162 (and the proximal coiled portion 130 coupled thereto). The proximal ends of the proximal stretch-resistant member 132 are terminated near the link 162 adjacent the distal aperture 165 of the link 162, mechanically coupling the link 162 (and the delivery assembly 150 coupled thereto) to the proximal coiled portion 130 of the vaso-occlusive device 110. The delivery junction 160 also includes an adhesive drop 166, which permeates the proximal end of the vaso-occlusive device 110 through the open pitch proximal windings 134, and binds together: (1) the hook 154 of the delivery assembly 150; (2) the link 162; (3) the proximal ends of the proximal stretch-resistant member 132; and (4) the open proximal end of the proximal coiled portion 130 of the vaso-occlusive device 110, thereby forming the delivery junction 160.

The proximal intra-device junction 170 includes a distal end of the proximal coiled portion 130 of the vaso-occlusive device 110 and a necked down proximal end of the braided portion 120 of the vaso-occlusive device 110. The proximal end of the braided portion 120 is necked down so that it fits inside of the open distal end of the proximal coiled portion 130. The proximal intra-device junction 170 also includes an adhesive drop 176, which permeates the coiled open distal end of proximal coiled portion 130 of the vaso-occlusive device 110. The adhesive 176 binds together: (1) the open distal end of the proximal coiled portion 130 of the vaso-occlusive device 110; (2) the distal ends of the proximal stretch-resistant member 132; and (3) the proximal end of the braided portion 120 of the vaso-occlusive device 110, thereby forming the proximal intra-device junction 170.

The distal intra-device junction 180 is a mirror image of the proximal intra-device junction 170. The distal intra-device junction 180 includes a necked down distal end of the braided portion 120 of the vaso-occlusive device 110 and an open proximal end of the distal coiled portion 140 of the vaso-occlusive device 110. The distal end of the braided portion 120 is necked down so that it fits inside of the open proximal end of the distal coiled portion 140. The distal intra-device junction 180 also includes an adhesive drop 186, which permeates the coiled open proximal end of the distal coiled portion 140 of the vaso-occlusive device 110. The adhesive 186 binds together: (1) the distal end of the braided portion 120 of the vaso-occlusive device 110; (2) the proximal ends of a distal stretch-resistant member 142; and (3) the open proximal end of the distal coiled portion 140 of the vaso-occlusive device 110, thereby forming the distal intra-device junction 180.

The distal coiled portion 140 also has an atraumatic distal tip 144, which may be formed from a small amount of adhesive. Distal ends of the distal stretch-resistant member 142 extend from the distal intra-device junction 180, through the distal coiled portion 140, and to the atraumatic distal tip 144, wherein the distal ends of the distal stretch-resistant member 142 are terminated within the atraumatic distal tip 144.

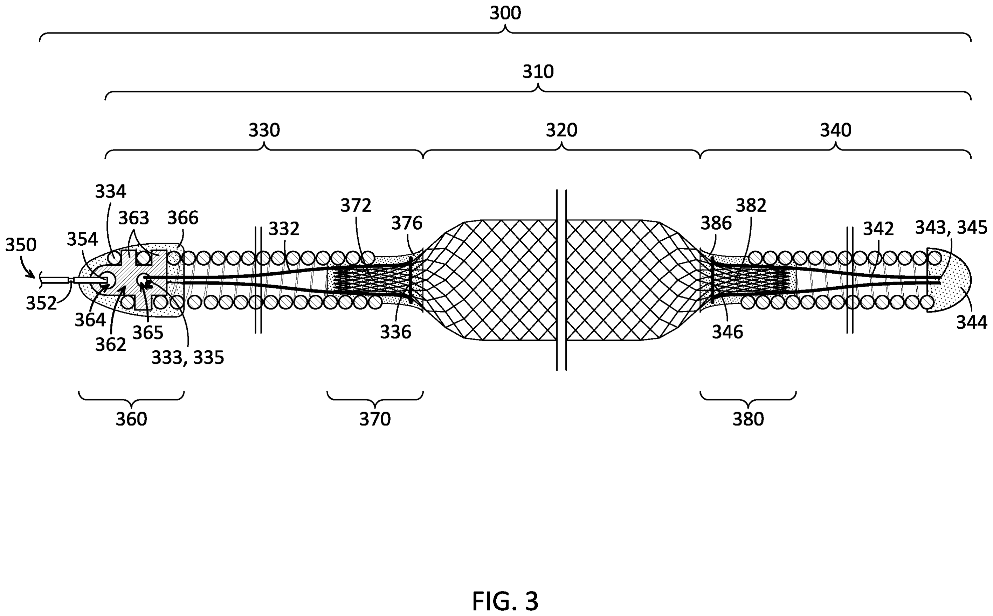

Figure 3 depicts a vaso-occlusive treatment system 300 according to various aspects of the disclosure. The vaso-occlusive treatment system 300 is similar to the vaso-occlusive treatment system 100 depicted in Figure 1. The main difference between the vaso-occlusive treatment systems 100, 300 is that the proximal and distal stretch-resistant members 332, 342 in vaso-occlusive treatment system 300 are external stretch-resistant members instead of extensions of braid wires from the braided portion 320. External stretch-resistant members can be selected with physical characteristics (e.g., diameter, strength, flexibility, etc.) independent of the braid wires in the braided portion 320. Accordingly, the external stretch-resistant members can be selected to tune the physical characteristics of various portions of the vaso-occlusive treatment system 300 (e.g., delivery junction 360, proximal coiled portion 330, proximal intra-device junction 370, distal intra-device junction 380, and distal coiled portion 340) independent of the braid wires in the braided portion 320.

The vaso-occlusive treatment system 300 includes a vaso-occlusive device 310 coupled to a delivery assembly 350 by a delivery junction 360. The delivery assembly 350 includes an electrolytically degradable segment 352 at a distal end thereof. The vaso-occlusive device 310 includes a braided portion 320 coupled to proximal and distal coiled portions 330, 340 by proximal and distal intra-device junctions 370, 380, respectively. The braided portion 320 can be braided from DFT (i.e., composite) wires or other alloy wires as described above, and has a substantially constant width (i.e., cross-sectional dimension). The proximal and distal coiled portions 330, 340 are respective coils wound from one or more DFT wires or other alloy wires as described above. According to various aspects of the disclosure, the braided and proximal and distal coiled portions 320, 330, 340 can be formed from other elongate members. The proximal coiled portion 330 also has a proximal stretch-resistant member 332, which extends distally from the delivery junction 360, through the proximal coiled portion 330, to the proximal intra-device junction 370, and reduces stretching of the proximal coil portion 330 of the vaso-occlusive device 310 as it is withdrawn proximally during delivery.

The proximal stretch-resistant member 332 is an external stretch-resistant member, which can be made from DFT (i.e., composite) wires or other elongated members. The proximal stretch-resistant member 332 has first and second ends 333, 335 and a bend 336 between the first and second ends 333, 335.

The delivery junction 360 includes a link/adapter 362 coupled to both the distal end of the delivery assembly 350 and the open proximal end of the proximal coiled portion 330, thereby coupling the delivery assembly 350 and the proximal coiled portion 330. The link 362 may be formed from a sheet, as described in U.S. Pat. No. 8,202,292, which were previously incorporated herein by reference herein. The link 362 is generally flat, and includes a plurality of fingers 363 formed in a proximal end thereof. The link also includes proximal and distal apertures 364, 365. The proximal coiled portion 330 of the vaso-occlusive device 310 is a coil with open pitch proximal windings 334 at the open proximal end thereof. At least some of these proximal windings 334 interlace with the fingers 363 of the link 362, coupling the link 362 to the proximal coiled portion 330 of the vaso-occlusive device 310.

The delivery junction 360 also includes a hook 354 formed from a distal end of a core wire of the delivery assembly 350, as described above. The hook 354 passes through the proximal aperture 364 of the link 362, coupling the delivery assembly 350 and link 362 (and the proximal coiled portion 330 coupled thereto). The first and second ends 333, 335 of the proximal stretch-resistant member 332 are terminated near the link 362 adjacent the distal aperture 365 of the link 362, mechanically coupling the link 362 (and the delivery assembly 350 coupled thereto) to the proximal coiled portion 330 of the vaso-occlusive device 310. The delivery junction 360 also includes an adhesive drop 366, which permeates the proximal end of the vaso-occlusive device 310 through the open pitch proximal windings 334, and binds together: (1) the hook 354 of the delivery assembly 350; (2) the link 362; (3) the first and second ends 333, 335 of the proximal stretch-resistant member 332; and (4) the open proximal end of the proximal coiled portion 330 of the vaso-occlusive device 310, thereby forming the delivery junction 360.

The proximal intra-device junction 370 includes a distal end of the proximal coiled portion 330 of the vaso-occlusive device 310 and a necked down proximal end of the braided portion 320 of the vaso-occlusive device 310. The proximal end of the braided portion 320 is necked down to form a packed proximal end bundle 372, which fits inside of the open distal end of the proximal coiled portion 330. The bend 336 of the proximal stretch-resistant member 332 loops around the proximal packed end bundle 372 and through openings in the proximal end of the braided portion 320, thereby mechanically securing the proximal stretch-resistant member 332 to the braided portions 320 of the vaso-occlusive device 310. The proximal intra-device junction 370 also includes an adhesive drop 376, which permeates the coiled open distal end of proximal coiled portion 330 of the vaso-occlusive device 310. The adhesive 376 binds together: (1) the open distal end of the proximal coiled portion 330 of the vaso-occlusive device 310; (2) the bend 336 of the proximal stretch-resistant member 332 around the packed proximal end bundle 372; and (3) the packed proximal end bundle 372 of the braided portion 320, thereby forming the proximal intra-device junction 370.

Similar to the proximal stretch-resistant member 332, the distal stretch-resistant member 342 is an external stretch-resistant member, which can be made from DFT (i.e., composite) wires or other elongated members. The distal stretch-resistant member 342 has first and second ends 343, 345 and a bend 346 between the first and second ends 343, 345.

The distal intra-device junction 380 is a mirror image of the proximal intra-device junction 370. The distal intra-device junction 380 includes a necked down distal end of the braided portion 320 of the vaso-occlusive device 310 and an open proximal end of the distal coiled portion 340 of the vaso-occlusive device 310. The distal end of the braided portion 320 is necked down to form a packed distal end bundle 382, which fits inside of the open proximal end of the distal coiled portion 340. The bend 346 of the distal stretch-resistant member 342 loops around the distal packed end bundle 382 and through openings in the distal end of the braided portion 320, thereby mechanically securing the distal stretch-resistant member 342 to the braided portions 320 of the vaso-occlusive device 310. The distal intra-device junction 380 also includes an adhesive drop 386, which permeates the coiled open proximal end of the distal coiled portion 340 of the vaso-occlusive device 310. The adhesive 376 binds together: (1) the packed distal end bundle 382 of the braided portion 320; (2) the bend 346 of the distal stretch-resistant member 342 around the packed distal end bundle 382; and (3) the open proximal end of the distal coiled portion 340 of the vaso-occlusive device 310, thereby forming the distal intra-device junction 380.

The distal coiled portion 340 also has an atraumatic distal tip 344, which may be formed from a small amount of adhesive. The first and second ends 343, 345 of the distal stretch-resistant member 342 extend from the distal intra-device junction 380, through the distal coiled portion 340, and to the atraumatic distal tip 344, wherein the first and second ends 343, 345 of the distal stretch-resistant member 342 are terminated within the atraumatic distal tip 344.

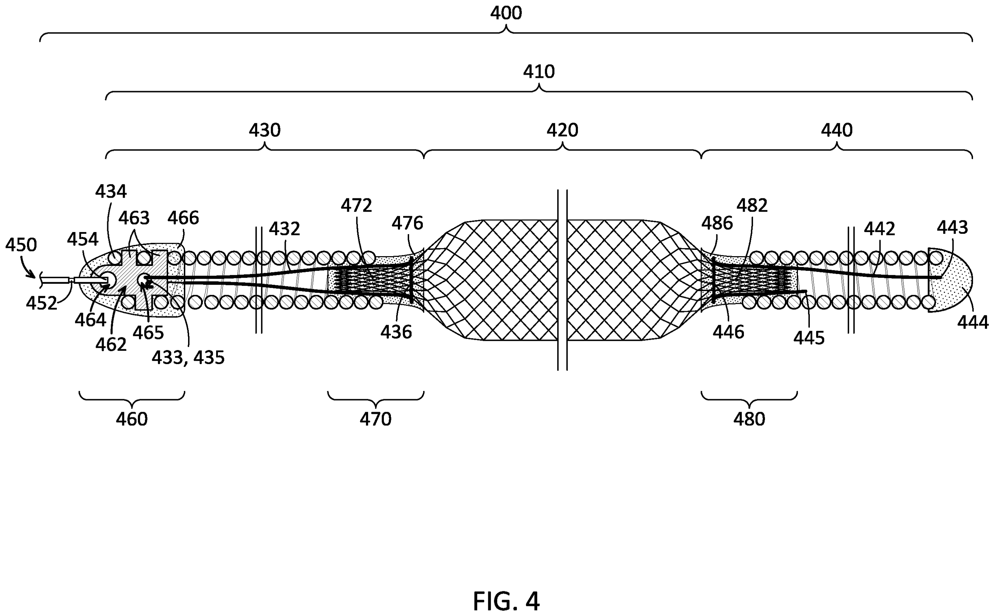

Figure 4 depicts a vaso-occlusive treatment system 400 according to various aspects of the disclosure. The vaso-occlusive treatment system 400 is similar to the vaso-occlusive treatment system 300 depicted in Figure 3, and similar elements have similar reference numbers. The main difference between the vaso-occlusive treatment systems 300, 400 is that the second end 445 of the distal stretch-resistant members 442 in vaso-occlusive treatment system 400 ends in the middle of the distal coiled portion 440 instead of reaching all the way distally to the atraumatic distal tip 444 at the distal end of the distal coiled portion 440.

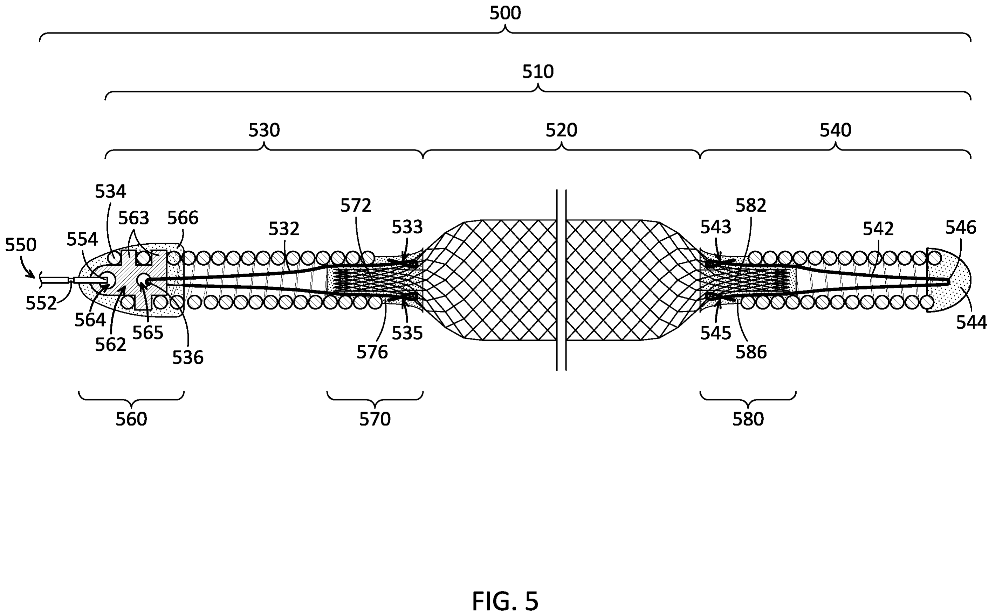

Figure 5 depicts a vaso-occlusive treatment system 500 according to various aspects of the disclosure. The vaso-occlusive treatment system 500 is similar to the vaso-occlusive treatment system 300 depicted in Figure 3, and similar elements have similar reference numbers. Like the delivery junction 360 in Figure 3, the delivery junction 560 includes a hook 554 formed from a distal end of a core wire of the delivery assembly 550, as described above. The hook 554 passes through the proximal aperture 564 of the link 562, coupling the delivery assembly 550 and link 562 (and the proximal coiled portion 530 coupled thereto). One difference between the vaso-occlusive treatment systems 300, 500 is that the proximal stretch-resistant member 532 is threaded through the proximal aperture 565 of the link 562 such that the bend 536 in the proximal stretch-resistant member 532 is disposed in the proximal aperture 565, mechanically coupling the link 562 (and the delivery assembly 550 coupled thereto) to the proximal coiled portion 530 of the vaso-occlusive device 510. The delivery junction 560 also includes an adhesive drop 566, which permeates the proximal end of the vaso-occlusive device 510 through the open pitch proximal windings 534, and binds together: (1) the hook 554 of the delivery assembly 550; (2) the link 562; (3) the bend 536 of the proximal stretch-resistant member 532; and (4) the open proximal end of the proximal coiled portion 530 of the vaso-occlusive device 510, thereby forming the delivery junction 560.

The proximal intra-device junction 570 includes a distal end of the proximal coiled portion 530 of the vaso-occlusive device 510 and a necked down proximal end of the braided portion 520 of the vaso-occlusive device 510. The proximal end of the braided portion 520 is necked down to form a packed proximal end bundle 572, which fits inside of the open distal end of the proximal coiled portion 530. Another difference between the vaso-occlusive treatment systems 300, 500 is that the first and second ends 533, 535 of the proximal stretch-resistant member 532 in vaso-occlusive treatment system 500 are each terminated adjacent (e.g., locked onto) the proximal end of the braided portion 520 distal of the proximal packed end bundle 572 instead of forming a bend 336 as in the vaso-occlusive treatment system 300. The proximal intra-device junction 570 also includes an adhesive drop 576, which permeates the coiled open distal end of proximal coiled portion 530 of the vaso-occlusive device 510. The adhesive 576 binds together: (1) the open distal end of the proximal coiled portion 530 of the vaso-occlusive device 510; (2) the first and second ends 533, 535 of the proximal stretch-resistant member 532 around the packed proximal end bundle 572 of the braided portion 520; and (3) the packed proximal end bundle 572, thereby forming the proximal intra-device junction 570.

Similar to the proximal stretch-resistant member 532, the distal stretch-resistant member 542 is an external stretch-resistant member, which can be made from DFT (i.e., composite) wires or other elongated members. The distal stretch-resistant member 542 has first and second ends 543, 545 and a bend 546 between the first and second ends 543, 545.

The distal intra-device junction 580 is a mirror image of the proximal intra-device junction 570. The distal intra-device junction 580 includes a necked down distal end of the braided portion 520 of the vaso-occlusive device 510 and an open proximal end of the distal coiled portion 540 of the vaso-occlusive device 510. The distal end of the braided portion 520 is necked down to form a packed distal end bundle 582, which fits inside of the open proximal end of the distal coiled portion 540. The first and second ends 543, 545 of the distal stretch-resistant member 542 in vaso-occlusive treatment system 500 are each terminated adjacent (e.g., locked onto) the distal end of the braided portion 520 proximal of the distal packed end bundle 582, thereby mechanically securing the distal stretch-resistant member 542 to the braided portions 520 of the vaso-occlusive device 510. The distal intra-device junction 580 also includes an adhesive drop 586, which permeates the coiled open proximal end of the distal coiled portion 540 of the vaso-occlusive device 510. The adhesive 586 binds together: (1) the packed distal end bundle 582 of the braided portion 520; (2) the first and second ends 543, 545 of the distal stretch-resistant member 542 around the packed distal end bundle 582; and (3) the open proximal end of the distal coiled portion 540 of the vaso-occlusive device 510, thereby forming the distal intra-device junction 580.

The distal coiled portion 540 also has an atraumatic distal tip 544, which may be formed from a small amount of adhesive. The distal stretch-resistant member 542 extends from the distal intra-device junction 580, through the distal coiled portion 540, and to the atraumatic distal tip 544, wherein the bend 546 in the distal stretch-resistant member 542 is disposed in the atraumatic distal tip 544 at the distal end of the distal coiled portion 540.

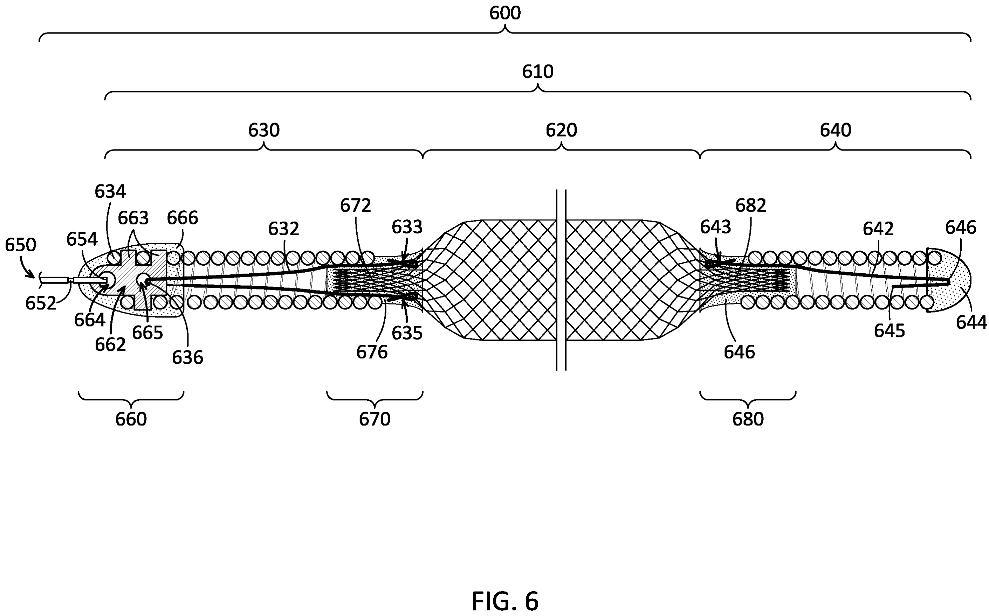

Figure 6 depicts a vaso-occlusive treatment system 600 according to various aspects of the disclosure. The vaso-occlusive treatment system 600 is similar to the vaso-occlusive treatment system 500 depicted in Figure 5, and similar elements have similar reference numbers. The main difference between the vaso-occlusive treatment systems 500, 600 is that the second end 645 of the distal stretch-resistant members 642 in vaso-occlusive treatment system 600 ends in the middle of the distal coiled portion 640 instead of reaching all the way proximally to the braided portion 620.

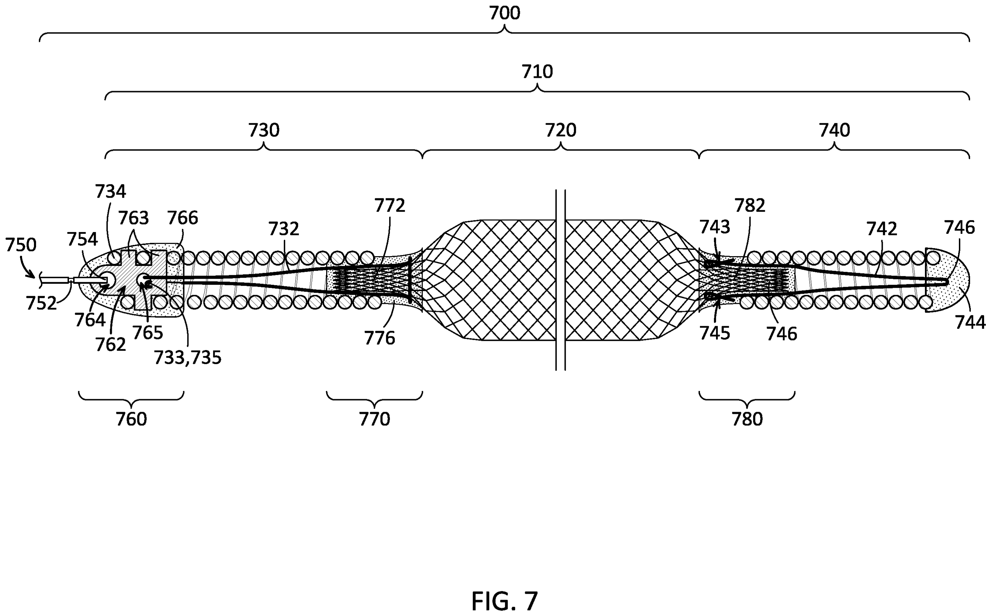

Figure 7 depicts a vaso-occlusive treatment system 700 according to various aspects of the disclosure. The vaso-occlusive treatment system 700 is similar to the vaso-occlusive treatment system 300 depicted in Figure 3, and similar elements have similar reference numbers. The main difference between the vaso-occlusive treatment systems 300, 700 is that the first and second ends 743, 745 of the distal stretch-resistant member 742 in vaso-occlusive treatment system 700 are each terminated adjacent (e.g., locked onto) the distal end of the braided portion 720 proximal of the distal packed end bundle 782. Further, the bend 746 in the distal stretch-resistant member 742 is disposed in the atraumatic distal tip 744 at the distal end of the distal coiled portion 740.

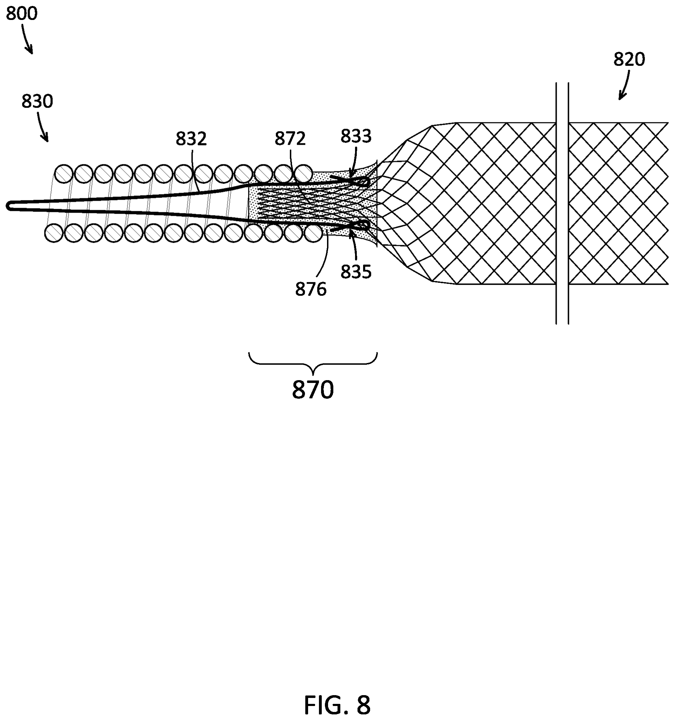

Figure 8 depicts portions of a vaso-occlusive treatment system 800 according to various aspects of the disclosure. Figure 8 focuses on the proximal intra-device junction 870 and the various components included therein (e.g., proximal coiled portion 830, proximal stretch-resistant member 832, braided portion 820, packed proximal end bundle 872, and adhesive 876). Proximal intra-device junction 870 is similar to the proximal intra-device junctions 570, 670 depicted in Figures 5 and 6. For instance, first and second ends 833, 835 of the proximal stretch-resistant member 832 in vaso-occlusive treatment system 800 are each terminated adjacent (e.g., locked onto) the proximal end of the braided portion 820 distal of the proximal packed end bundle 872. Each of the first and second ends 833, 835 are terminated adjacent (e.g., locked onto) the proximal end of the braided portion 820 by being threaded through the braided portion 820 and then bent back on itself.

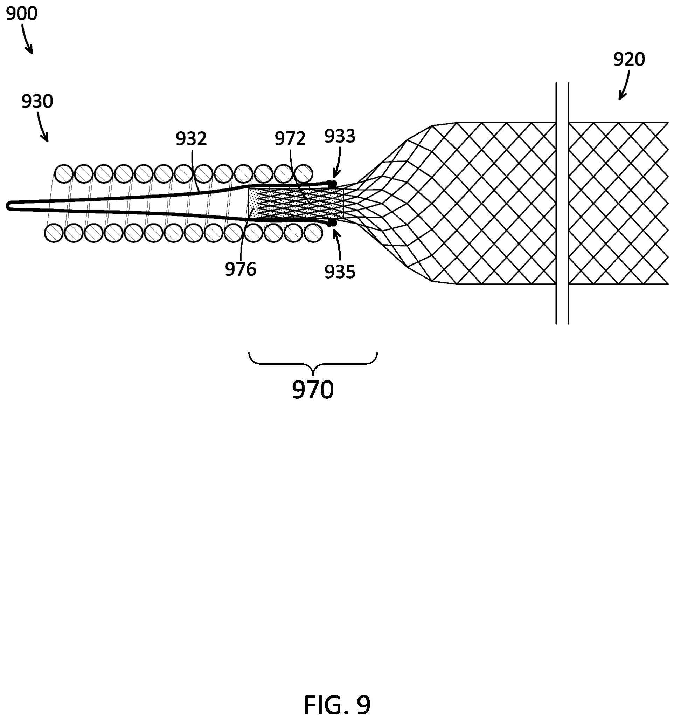

Figure 9 depicts portions of a vaso-occlusive treatment system 900 according to various aspects of the disclosure. Figure 9 focuses on the proximal intra-device junction 970 and the various components included therein (e.g., proximal coiled portion 930, proximal stretch-resistant member 932, braided portion 920, packed proximal end bundle 972, and adhesive 976). Proximal intra-device junction 970 is similar to the proximal intra-device junction 870 in Figure 8. The main difference between the proximal intra-device junctions 870, 970 is that first and second ends 933, 935 of the proximal stretch-resistant member 932 in vaso-occlusive treatment system 900 are each flattened against the braided portion 920 forming the proximal packed end bundle 972 to couple the proximal stretch-resistant member 932 to the braided portion 920.

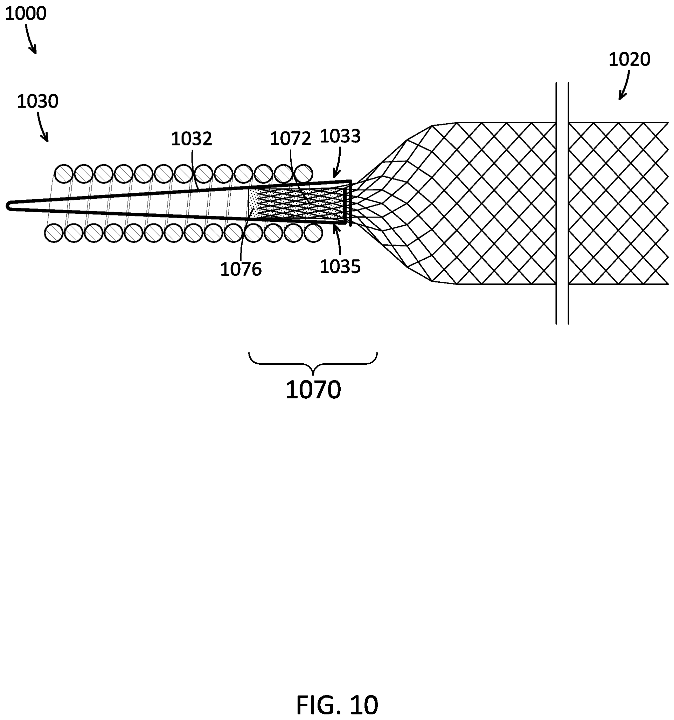

Figure 10 depicts portions of a vaso-occlusive treatment system 1000 according to various aspects of the disclosure. Figure 10 focuses on the proximal intra-device junction 1070 and the various components included therein (e.g., proximal coiled portion 1030, proximal stretch-resistant member 1032, braided portion 1020, packed proximal end bundle 1072, and adhesive 1076). Proximal intra-device junction 1070 is similar to the proximal intra-device junction 870 in Figure 8. The main difference between the proximal intra-device junctions 870, 1070 is that first and second ends 1033, 1035 of the proximal stretch-resistant member 1032 in vaso-occlusive treatment system 1000 are each are terminated adjacent (e.g., locked onto) the proximal end of the braided portion 1020 by being threaded through the braided portion 1020 and then crossing the other end to couple the proximal stretch-resistant member 1032 to the braided portion 1020.

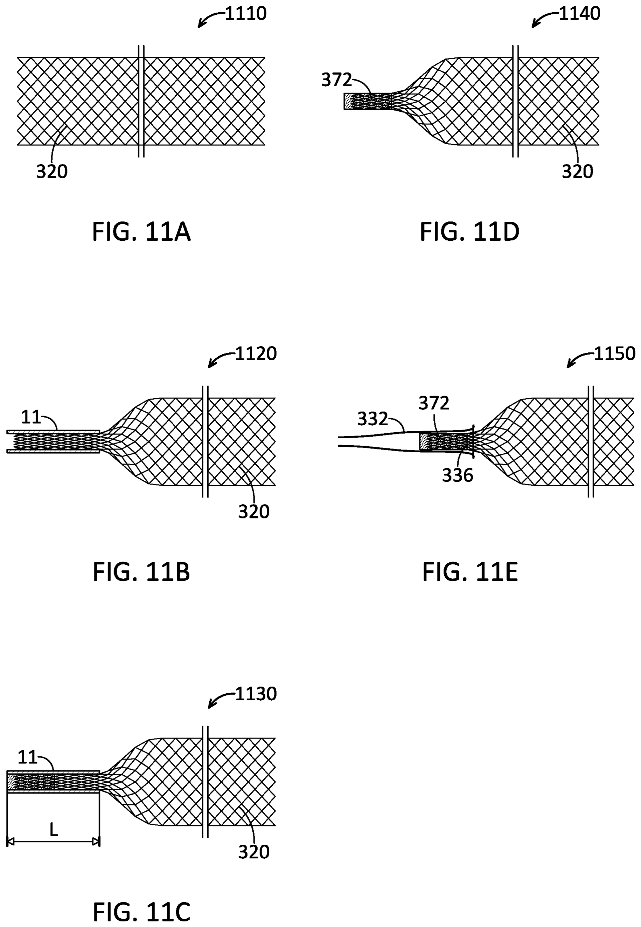

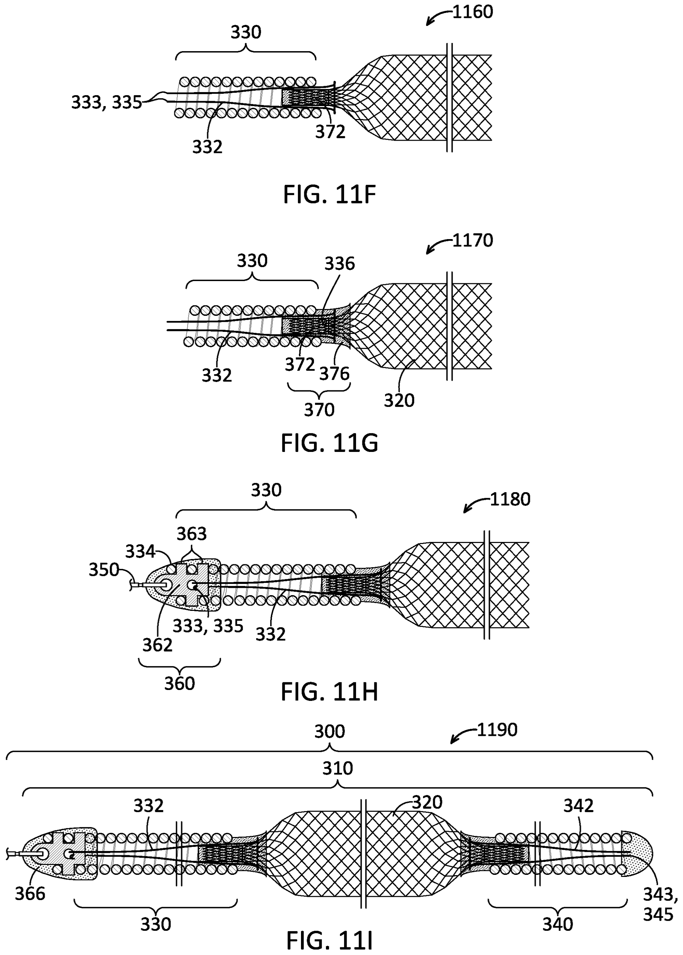

Figures 11A to 11I schematically depict a method for manufacturing a vaso-occlusive treatment system including a vaso-occlusive device such as the vaso-occlusive treatment system 300 and vaso-occlusive device 310 depicted in Figure 3. Figure 11A depicts step 1110, in which the braided portion 320 is cut to length after secondary winding. Figure 11B depicts step 1120 in which a heat-shrink polymer tube 11 is disposed around a proximal end of the braided portion 320 and heat is applied to the heat-shrink polymer tube 11 to compress the proximal end of the braided portion 320. An example of such a heat-shrink polymer tube 11 is PTFE heat shrinking tubing. Figure 11C depicts step 1130 in which adhesive is applied to the proximal end of the compressed braided portion 320. For example, adhesive may be applied from the proximal end of the compressed braided portion 320 into the heat-shrink polymer tube 11, along a desired length "L". Figure 11D depicts step 1140 in which the shrunk polymer tube (not shown) is removed from around the compressed proximal end of the braided portion 320 to form the proximal packed braid end bundle 372. Figure 11E depicts step 1150 in which the external stretch-resistant member 332 is threaded through the proximal end of the braided portion 320 distal of the packed end bundle 372 to form the bend 336 through the proximal end of the braided portion 320 distal of the packed end bundle 372. Figure 11F depicts step 1160 in which first end 333 and second end 335 of the external stretch-resistant member 332 are threaded proximally into the proximal coil portion 330 along with at least a portion of the proximal end of the proximal packed braid end bundle 372. Figure 11G depicts step 1170 in which adhesive 376 is applied to the distal end of the proximal coiled portion 330, the proximal packed braid end bundle 372, the bend 336 in the external stretch-resistant member 332, and the proximal end of the braided portion 320 to form the proximal intra-device junction 370. Figure 11H depicts step 1180 in which the first and second ends 333, 335 of the external stretch-resistant member 332 are coupled to the link 362 connected to the delivery wire assembly 350 and the proximal end 334 of the proximal coiled portion 330 is threaded onto the fingers 363 of the link 362. Adhesive 366 is then applied to the distal end of delivery assembly 350, the link 363, the proximal end of the proximal coiled portion 330, and the first and second ends 333, 335 of the external stretch-resistant member 332 to complete assembly of the delivery junction 360. Figure 11I depicts step 1190 in which steps 11B to 11G are repeated with the distal end of the braided portion 320 and adhesive is applied to the distal end of the distal coiled portion 340 to secure first and second ends 343, 345 of the distal external stretch-resistant member 342 to complete assembly of the vaso-occlusive treatment system 300 and vaso-occlusive device 310.

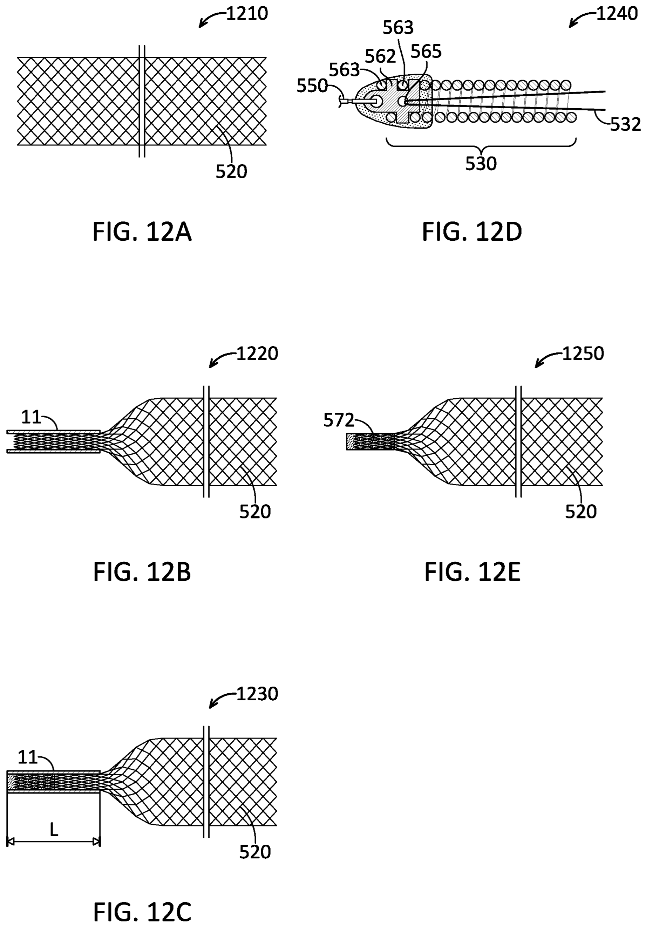

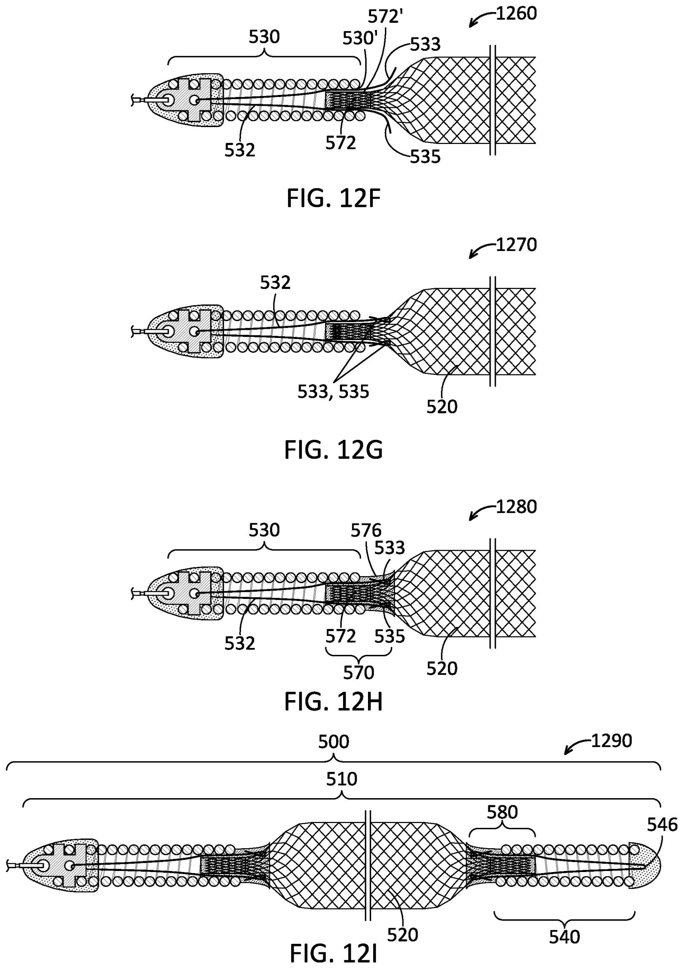

Figures 12A to 12I schematically depict a method for manufacturing a vaso-occlusive treatment system including a vaso-occlusive device such as the vaso-occlusive treatment system 500 and vaso-occlusive device 510 depicted in Figure 5. Figure 12A depicts step 1210 in which the braided portion 520 is cut to length after secondary winding. Figure 12B depicts step 1220 in which a heat-shrink polymer tube 11 is disposed around a proximal end of the braided portion 520 and heat is applied to the heat-shrink polymer tube 11 to compress the proximal end of the braided portion 520. An example of a heat-shrink polymer tube 11 is PTFE heat shrinking tubing. Figure 12C depicts step 1230 in which adhesive is applied to the proximal end of the compressed braided portion 520. For example, adhesive may be applied from the proximal end of the compressed braided portion 520 into the heat-shrink polymer tube 11, along desired length "L". Figure 12D depicts step 1240 in which the external stretch-resistant member 532 is threaded through the proximal aperture 565 of the link 562 connected to the delivery wire assembly 550 and the proximal end of the proximal coiled portion 530 is threaded onto the fingers 563 of the link 562. Figure 12E depicts step 1250 in which the shrunk polymer tube (not shown) is removed from around the compressed proximal end of the braided portion 520 to form a proximal packed braid end bundle 572. Figure 12F depicts step 1260 in which the proximal end of the proximal packed braid end bundle 572 is inserted into the distal end of the proximal coiled portion 530 while first and second ends 533, 535 of the external stretch-resistant member 532 are maintained between an external/outer surface 572' of the proximal packed braid end bundle 572 and an internal/inner surface 530' of the distal end of the proximal coiled portion 530. Figure 12G depicts step 1270 in which first and second ends 533, 535 of the external stretch-resistant member 532 are terminated adjacent (e.g., locked onto) the proximal end of the braided portion 520 and trimmed. Each of the first and second ends 533, 535 are terminated adjacent (e.g., locked onto) the proximal end of the braided portion 520 by being threaded through the braided portion 520 and then bent back on itself. Figure 12H depicts step 1280 in which adhesive 576 is applied to the distal end of the proximal coiled portion 530, the proximal packed braid end bundle 572, the first and second ends 533, 535 of the external stretch-resistant member 532, and the proximal end of the braided portion 520 to form the proximal intra-device junction 570. Figure 12I depicts step 1290 in which steps 12B to 12G are repeated with the distal end of the braided portion 520 to form the distal intra-device junction 580 and adhesive is applied to the distal end of the distal coiled portion 540 to secure the bend 546 in the distal external stretch-resistant member 542 to complete assembly of the vaso-occlusive treatment system 500 and vaso-occlusive device 510.

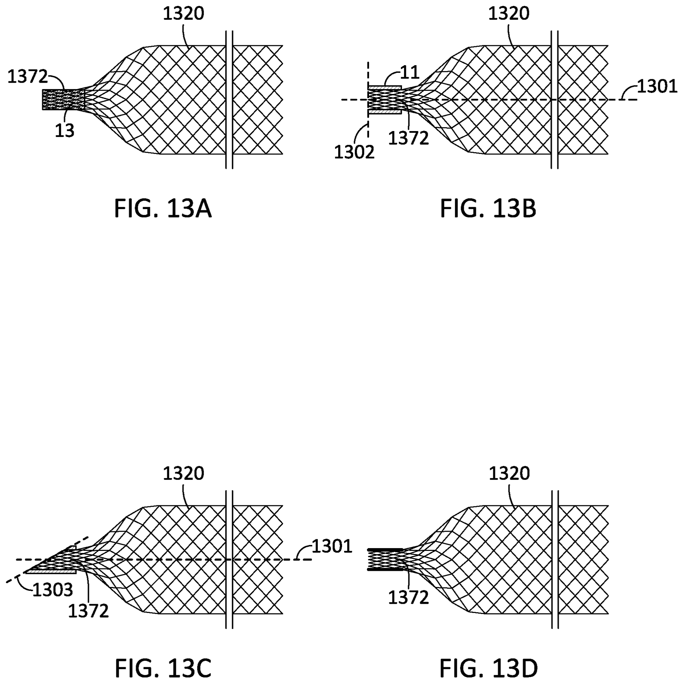

Figures 13A to 13D depict proximal packed braid end bundles according to various aspects of the disclosure. Figure 13A depicts a proximal packed braid end bundle 1372 formed from a compressed proximal end of a braided portion 1320 and adhesive 13 such as the proximal packed braid end bundles described herein. Figure 13B depicts a proximal packed braid end bundle 1372 that is held in place by a heat-shrink tube 11 surrounding a compressed proximal end of the braided portion 1320 without the addition of adhesive/glue. The proximal end of the proximal packed end bundle 1372 has a surface 1302 approximately orthogonal to a longitudinal axis 1301 of the braided portion 1320. Figure 13C also depicts a proximal packed braid end bundle 1372 with a proximal end surface 1303 oblique or at an angle with respect to the longitudinal axis 1301 of the braided portion 1320. Such an oblique surface 1303 facilitates insertion of the proximal packed end bundle 1372 into a coiled portion. Figure 13D depicts the proximal packed end bundle 1372 formed from a compressed and welded proximal end of the braided portion 1320.

The external stretch-resistant member can have any shape and any form. For example, the external stretch-resistant member can be a wire with various cross-sectional shapes, or a tube. The external stretch-resistant member can be made from any type of biocompatible materials. The external stretch-resistant member can be made from the same material as the braid wires or different materials. For example, it can be a DFT with a composition as described above. The external stretch-resistant member can also be made from a metal or an alloy such as platinum (Pt), tungsten (W), gold (Au), platinum group metals, and their alloys. For example, the external stretch resistant member can be made from Pt-8%wtW (Pt-8W) or Au-34%wtPt (AuPt34). The delivery junction manufactured using the external stretch-resistant members described herein can have improved junction tensile strength. According to various aspects of the disclosure, external stretch-resistant members made from AuPt34 with a diameter of 0.0011 in. incorporated into delivery junction such as those described herein resulted in delivery junction tensile strengths of 0.350 lb. ± 0.025 lb. The incorporation of external stretch-resistant members such as those described herein into vaso-occlusive devices and vaso-occlusive treatment systems can also simplify assembly (especially of intra-device junctions), and can allow delivery junction tensile strength to be controlled independent of braid wire properties or braid wire diameter.

Various aspects of the disclosure are described hereinafter with reference to the figures. It should be noted that the figures are not drawn to scale and that elements of similar structures or functions are represented by like reference numerals throughout the figures. It should also be noted that the figures are only intended to facilitate the description, and are not intended as an exhaustive description of the disclosure or as a limitation on the scope of the disclosure, which is defined only by the appended claims and their equivalents. In addition, the respective illustrated aspects need not each have all the features or advantages of aspects described herein. A feature or an advantage described in conjunction with a particular aspect of the disclosure is not necessarily limited to that aspect and can be practiced with any other aspect even if not so illustrated.
The following clauses set out aspects of the present disclosure:
1. A vaso-occlusive treatment system, comprising:
   a delivery assembly; and
   a vaso-occlusive device detachably coupled to the delivery assembly by a delivery assembly junction, the vaso-occlusive device comprising a braided portion formed out of one or more wires, the braided portion comprising a packed end bundle, a coiled portion coupled to the braided portion, and an intra-device junction coupling the braided portion to the coiled portion, the intra-device junction comprising a stretch-resistant member spanning from the packed end bundle to the delivery assembly junction.
2. The vaso-occlusive treatment system of clause 1, wherein the packed end bundle comprises an adhesive disposed at an end of the braided portion.
3. The vaso-occlusive treatment system of any of clause 1, wherein the packed end bundle comprises a weld disposed at an end of the braided portion.
4. The vaso-occlusive treatment system of any of clauses 1 to 3, wherein the packed end bundle defines an end surface that is orthogonal to a longitudinal axis of the packed end bundle.
5. The vaso-occlusive treatment system of any of clauses 1 to 3, wherein the packed end bundle defines an end surface that is oblique to a longitudinal axis of the packed end bundle.
6. The vaso-occlusive treatment system of any of clauses 1 to 5, wherein the stretch-resistant member comprises first and second ends.
7. The vaso-occlusive treatment system of clause 6, wherein the first end of the stretch-resistant member terminates in the delivery wire junction, and wherein the second end of the stretch-resistant member terminates within the braided portion or the coiled portion.
8. The vaso-occlusive treatment system of clauses 6 or 7, wherein the first and second ends are flattened.
9. The vaso-occlusive treatment system of clause 6, wherein the first and second ends are each terminated within the braided portion.
10. The vaso-occlusive treatment system of clause 6, wherein the first and second ends cross each other within the braided portion.
11. The vaso-occlusive treatment system of clause 6, wherein the stretch-resistant member further comprises a bend.
12. The vaso-occlusive treatment system of clause 11, wherein the first and second ends terminate within the delivery wire junction, and wherein the bend is disposed adjacent the packed end bundle.
13. The vaso-occlusive treatment system of clause 12, wherein the bend is disposed distal of the packed end bundle.
14. The vaso-occlusive treatment system of clause 11, wherein the first and second ends terminate adjacent the packed end bundle, and wherein the bend is disposed within the delivery wire junction.
15. The vaso-occlusive treatment system of clause 14, wherein the delivery assembly junction comprises a link defining an opening, and wherein the bend is disposed in the opening.
16. The vaso-occlusive treatment system of clauses 14 or 15, wherein each of the first and second ends is terminated within the braided portion.
17. The vaso-occlusive treatment system of clause 6, further comprising a second stretch-resistant member, wherein the second stretch-resistant member comprises third and fourth ends.
18. The vaso-occlusive treatment system of clause 17, wherein the first and third ends terminate within the delivery wire junction, and wherein the second and fourth ends terminate adjacent the packed end bundle.
19. The vaso-occlusive treatment system of any of clauses 1 to 5, wherein the stretch-resistant member is a loop comprising first and second bends.
20. The vaso-occlusive treatment system of clause 19, wherein the first bend is disposed within the delivery wire junction, and wherein the second bend is disposed adjacent the packed end bundle.
21. The vaso-occlusive treatment system of clause 20, wherein the first bend is disposed distal of the packed end bundle.
22. The vaso-occlusive treatment system of clauses 20 or 21, wherein the delivery assembly junction comprises a link defining an opening, and wherein the second bend is disposed in the opening.
23. The vaso-occlusive treatment system of any of clauses 1 to 44, wherein the packed end bundle is a proximal end bundle, wherein the coiled portion is a proximal coiled portion, wherein the intra-device junction is a proximal intra-device junction, wherein the stretch-resistant member is a proximal stretch-resistant member, wherein the braided portion further comprises a distal packed end bundle, wherein the system further comprises:
   a distal coiled portion coupled to the distal packed end bundle; and a distal intra-device junction coupling the braided portion to the distal coiled portion, the distal intra-device junction comprising a distal stretch-resistant member spanning from the distal packed end bundle to a distal end of the distal coiled portion.
24. The vaso-occlusive treatment system of clause 23, wherein the distal packed end bundle comprises an adhesive disposed at a distal end of the braided portion.
25. The vaso-occlusive treatment system of any of clause 23, wherein the distal packed end bundle comprises a weld disposed at a distal end of the braided portion.
26. The vaso-occlusive treatment system of any of clauses 23 to 25, wherein the distal packed end bundle defines a distal end surface that is orthogonal to a longitudinal axis of the distal packed end bundle.
27. The vaso-occlusive treatment system of any of clauses 23 to 25, wherein the distal packed end bundle defines a distal end surface that is oblique to a longitudinal axis of the distal packed end bundle.
28. The vaso-occlusive treatment system of any of clauses 23 to 27, wherein the distal stretch-resistant member comprises first and second distal stretch-resistant member ends.
29. The vaso-occlusive treatment system of clause 28, wherein the first distal stretch-resistant member end of the distal stretch-resistant member terminates in the distal end of the distal coiled portion, and wherein the second distal stretch-resistant member end of the distal stretch-resistant member terminates within the braided portion or the distal coiled portion.
30. The vaso-occlusive treatment system of clauses 28 or 29, wherein the first and second distal stretch-resistant member ends are flattened.
31. The vaso-occlusive treatment system of clause 28, wherein the first and second distal stretch-resistant member ends are each terminated within the braided portion.
32. The vaso-occlusive treatment system of clause 28, wherein the first and second distal stretch-resistant member ends cross each other within the braided portion.
33. The vaso-occlusive treatment system of clause 28, wherein the distal stretch-resistant member further comprises a distal stretch-resistant member bend.
34. The vaso-occlusive treatment system of clause 33, wherein the first and second distal stretch-resistant member ends terminate within the distal end of the distal coiled portion, and wherein the distal stretch-resistant member bend is disposed adjacent the distal packed end bundle.
35. The vaso-occlusive treatment system of clause 34, wherein the distal stretch-resistant member bend is disposed proximal of the distal packed end bundle.
36. The vaso-occlusive treatment system of clause 33, wherein the first and second distal stretch-resistant member ends terminate adjacent the distal packed end bundle, and wherein the distal stretch-resistant member bend is disposed within the distal end of the distal coiled portion.
37. The vaso-occlusive treatment system of clause 36, wherein each of the first and second distal stretch-resistant member ends is terminated within the braided portion.
38. The vaso-occlusive treatment system of clause 28, further comprising a second distal stretch-resistant member, wherein the second distal stretch-resistant member comprises third and fourth distal stretch-resistant member ends.
39. The vaso-occlusive treatment system of clause 38, wherein the first and third distal stretch-resistant member ends terminate within the distal end of the distal coiled portion, and wherein the second and fourth distal stretch-resistant member ends terminate adjacent the distal packed end bundle.
40. The vaso-occlusive treatment system of any of clauses 23 to 27, wherein the distal stretch-resistant member is a loop comprising first and second distal stretch-resistant member bends.
41. The vaso-occlusive treatment system of clause 40, wherein the first distal stretch-resistant member bend is disposed within the distal end of the distal coiled portion, and wherein the second distal stretch-resistant member bend is disposed adjacent the distal packed end bundle.
42. The vaso-occlusive treatment system of clause 41, wherein the first distal stretch-resistant member bend is disposed proximal of the distal packed end bundle.
43. The vaso-occlusive treatment system of any of clauses 1 to 42, the delivery assembly having a distal end, wherein the distal end forms a hook.
44. The vaso-occlusive treatment system of any of clauses 1 to 43, the delivery assembly junction comprising a link having a proximal end and a distal end, wherein the distal end of the link comprises a plurality of fingers.
45. A method of manufacturing a vaso-occlusive treatment system, comprising:
   forming a vaso-occlusive device, wherein forming the vaso-occlusive device comprises
      disposing a tubing around a proximal end of a braided portion formed from one or more wires,
      applying heat to the tubing to shrink the tubing around the proximal end of the braided portion to form a shrunken proximal end,
      applying glue to the shrunken proximal end to form a packed end bundle,
      removing the tubing from around the packed end bundle,
      threading a stretch-resistant member through the braided portion adjacent to and distal of the packed end bundle to form a bend of the stretch-resistant member in the braided portion adjacent to and distal of the packed end bundle,
      inserting the stretch-resistant member and at least a portion of the packed end bundle into a coiled portion, and
      applying glue to the packed end bundle and the bend of the stretch-resistant member to form an intra-device junction;
   coupling a distal end of a delivery assembly to a proximal end of the coiled portion and a proximal end of the stretch-resistant member; and
   applying glue to the distal end of the delivery assembly, the proximal end of the coiled portion, and the proximal end of the stretch-resistant member to form a delivery junction.
46. The method of clause 45, wherein the coiled portion is a proximal coiled portion, the method further comprising coupling a distal coiled portion to a distal end of the braided portion.
47. A method of manufacturing a vaso-occlusive treatment system, comprising:
   forming a packed end bundle, wherein forming the packed end bundle comprises
      disposing a tubing around a proximal end of a braided portion formed from one or more wires,
      applying heat to the tubing to shrink the tubing around the proximal end of the braided portion to form a shrunken proximal end,
      applying glue to the shrunken proximal end to form a packed end bundle, and
      removing the tubing from around the packed end bundle;
   forming a delivery junction, wherein forming the delivery junction comprises
      coupling a distal end of a link to a proximal end of a coiled portion,
      coupling a distal end of a delivery assembly to a proximal end of the link,
      threading a stretch-resistant member through an opening defined by the link to form a bend of the stretch-resistant member in the opening, and
      applying glue to the link, the proximal end of the coiled portion, the distal end of the delivery assembly, and the bend of the stretch-resistant member to form the delivery junction;
   inserting at least a portion of the packed end bundle into the coiled portion;
   coupling an end of the stretch-resistant member to the braided portion adjacent to and distal of the packed end bundle; and
   applying glue to the packed end bundle and the end of the stretch-resistant member to form an intra-device junction.
48. The method of clause 47, wherein the coiled portion is a proximal coiled portion, the method further comprising coupling a distal coiled portion to a distal end of the braided portion.

## Claims

1. A vaso-occlusive treatment system (300, 400), comprising:
a delivery assembly (350, 450); and
a vaso-occlusive device (310, 410) detachably coupled to the delivery assembly (350, 450) by a delivery assembly junction (360, 460), wherein the vaso-occlusive device (300, 400) comprises
a braided portion (320, 420) formed out of one or more wires, the braided portion (320, 420) comprising a packed end bundle (372, 472),
a coiled portion (330, 430) coupled to the braided portion (320, 420), and
an intra-device junction (370, 470) coupling the braided portion (320, 420) to the coiled portion (330, 430), the intra-device junction (370, 470) comprising a stretch-resistant member (332, 432) spanning from the packed end bundle (372, 472) to the delivery assembly junction (360, 460), wherein
the stretch-resistant member (332, 432) further comprising a bend (336, 436),
the delivery assembly junction (360, 460) comprising a link (362, 462) defining an opening (365, 465), and
the bend (336, 436) is looped around the proximal packed end bundle (372, 472) and through openings in the proximal end of the braided portion (320, 420),
the stretch-resistant member (332, 432) comprising a first end (333, 433) and a second end (335, 435),
wherein each of the first end (333, 433) and the second end (335, 435) is mechanically coupled to the link (362, 462) adjacent the opening (365, 465).

2. The vaso-occlusive treatment system (300, 400) of claim 1, wherein the packed end bundle (372, 472) is a proximal end bundle (372, 472),
wherein the coiled portion (330, 430) is a proximal coiled portion (330, 430),
wherein the intra-device junction (370, 470) is a proximal intra-device junction (370, 470),
wherein the stretch-resistant member (332, 432) is a proximal stretch-resistant member (332, 432),
wherein the braided portion (320, 420) further comprises a distal packed end bundle (382, 482),
wherein the system (300, 400) further comprises:
a distal coiled portion (340, 440) coupled to the distal packed end bundle (382, 482); and
a distal intra-device junction (380, 480) coupling the braided portion (320, 420) to the distal coiled portion (340, 440), the distal intra-device junction (380, 480) comprising a distal stretch-resistant member (342, 442) spanning from the distal packed end bundle (382, 482) to a distal end of the distal coiled portion (340, 440).

3. The vaso-occlusive treatment system (300, 400) of claim 2, wherein the distal stretch-resistant member (342, 442) comprises a distal first end (343, 443) and a distal second end (345, 445).

4. The vaso-occlusive treatment system (300, 400) of claim 3, further comprising an atraumatic distal tip (344, 444) formed of an adhesive.

5. The vaso-occlusive treatment system (300) of claim 4, wherein each of the distal first end (343) and the distal second end (345) of the distal stretch-resistant member (342) terminate within the atraumatic distal tip (344).

6. The vaso-occlusive treatment system (400) of claim 4, wherein the distal first end (443) of the distal stretch-resistant member (442) terminates within the atraumatic distal tip (444) and the distal second end (445) of the distal stretch-resistant member (442) does not terminate within the atraumatic distal tip (444).

7. The vaso-occlusive treatment system (300, 400) of any of claims 2-6, wherein the distal packed end bundle (382, 482) comprises an adhesive (386, 486) disposed at a distal end of the braided portion (320, 420).

8. The vaso-occlusive treatment system (300, 400) of any of claims 2-6, wherein the distal packed end bundle (382, 482) comprises a weld disposed at a distal end of the braided portion (320, 420).

9. The vaso-occlusive treatment system (300, 400) of any of claims 2 to 8, wherein the distal packed end bundle (382, 482) defines a distal end surface that is orthogonal to a longitudinal axis of the distal packed end bundle.

10. The vaso-occlusive treatment system (300, 400) of any of claims 2 to 8, wherein the distal packed end bundle (382, 482) defines a distal end surface that is oblique to a longitudinal axis of the distal packed end bundle.

11. The vaso-occlusive treatment system of (300, 400) any of claims 1 to 10, wherein the proximal end of the braided portion (320, 420) is disposed in a distal end of the coiled portion (330, 430).

12. The vaso-occlusive treatment system of (300, 400) any of claims 1 to 11, wherein the distal end of the braided portion (320, 420) is disposed in a proximal end of the distal coiled portion (340, 440).
